(19) Europäisches Patentamt
European Patent Office
Office européen des brevets

(11) Veröffentlichungsnummer: **0 045 426**
**A1**

# (12) EUROPÄISCHE PATENTANMELDUNG

(21) Anmeldenummer: **81105675.3**

(22) Anmeldetag: **20.07.81**

(51) Int. Cl.³: **C 07 C 51/00**
C 07 C 57/03, C 07 C 21/19
C 07 C 21/04, C 07 C 17/26
C 07 C 17/34, C 07 C 69/533

(30) Priorität: **01.08.80 DE 3029270**

(43) Veröffentlichungstag der Anmeldung:
**10.02.82 Patentblatt 82/6**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Arlt, Dieter, Prof. Dr.**
**Rybnikerstrasse 2**
**D-5000 Koeln 80(DE)**

(72) Erfinder: **Jautelat, Manfred, Dr.**
**Müllersbaum 28**
**D-5093 Burscheid(DE)**

(54) **Verfahren zur Herstellung von 3,5-Dimethyl-4-pentensäure.**

(57) Verfahren zur Herstellung von 3,3-Dimethyl-4-pentensäure der Formel

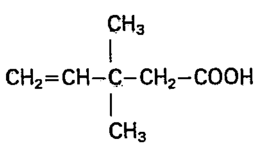

durch Umsetzung von Verbindungen der Formel

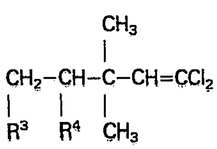

in welcher
$R^3$ für Chlor und
$R^4$ für Wasserstoff stehen; oder $R^3$ und $R^4$ gemeinsam eine Doppelbindung bestehen,
mit Alkoholaten der Formel

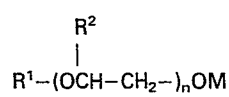

in welcher
$R^1$ für Wasserstoff, Alkyl oder M,
$R^2$ für Wasserstoff und Alkyl,
M für Alkalimetall und
n für eine ganze Zahl von 1 bis 6 steht,
bei Temperatur von 100 - 250°C und nachfolgende Hydrolyse.
1,1-Dichlor-3,3-dimethyl-1,4-pentadien und
3,3-Dimethyl-1,1,5-trichlor-1-penten.

EP 0 045 426 A1

0045426



BAYER AKTIENGESELLSCHAFT 5090 Leverkusen, Bayerwerk
Zentralbereich
Patente, Marken und Lizenzen Rt/kl-c
IVa / ZP

## Verfahren zur Herstellung von 3,5-Dimethyl-4-pentensäure

Die vorliegende Erfindung betrifft ein Verfahren zur Herstellung der bekannten 3,3-Dimethyl-4-pentensäure.

Es ist bereits bekannt, daß man 3,3-Dimethyl-4-pentensäure über eine 4-stufige Malonestersynthese aus 3-Chlor-3-methyl-1-butin erhält (J. Org. Chem. 27, 3602 (1962)). Der Nachteil dieses Verfahrens liegt in den teuren Ausgangsmaterialien und der unbefriedigenden Ausbeute von 50 % in der letzten Stufe.

Es ist weiter bekannt, diese Säuren durch alkalische Spaltung des Ammoniumsalzes

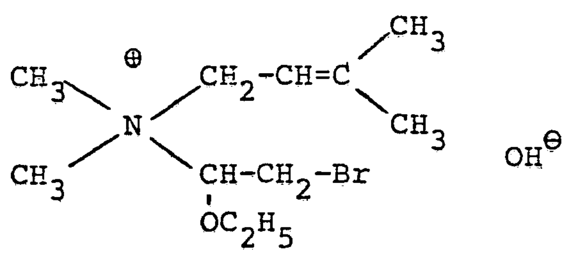



BAD ORIGINAL

herzustellen (Izv. Acad. Nauk. Arm. SSR, Khmi. Nauki 18, 572 (1965) und C.A. 65, 3725 f). Der Nachteil dieses Verfahrens liegt in der schwierigen Zugänglichkeit des Ausgangsmaterials.

Ein weiteres Verfahren geht von 3,3-Dimethyl-4-penten-1-al aus, das über Oxim und Nitril zur Säure führt (DOS 26 21 832). Auch dieses Verfahren ist für eine technische Ausführung durch die Vielstufigkeit und das teure Ausgangsmaterial uninteressant.

Der gleiche Mangel haftet auch dem Verfahren an, das über 4,4-Dimethyl-hex-5-en-2-on als Zwischenprodukt durch Haloformreaktion zur 3,3-Dimethyl-4-pentensäure führt (DOS 26 21 833).

Einen weiteren Zugang bietet das Verfahren aus 3-Methyl-2-buten-1-ol und Orthoessigester über eine Claisen-Umlagerung (DOS 25 39 895). Dieses Verfahren hat den Nachteil, daß die verwendeten Ausgangsmaterialien selbst in mehrstufigen Verfahren hergestellt werden müssen und nicht für jeden Produzenten verfügbar sind.

Es wurde nun gefunden, daß man 3,3-Dimethyl-4-pentensäure aus allgemein verfügbaren Grundchemikalien - nämlich Isopren, Vinylidenchlorid, Chlorwasserstoff und Alkalihydroxiden - nach einem neuartigen Verfahren herstellen kann.



BAD ORIGINAL

Die vorliegende Erfindung betrifft daher ein Verfahren zur Herstellung der 3,3-Dimethyl-4-pentensäure der Formel I

$$\begin{array}{c} \phantom{CH_2=CH-}CH_3 \\ CH_2=CH-\overset{|}{\underset{|}{C}}-CH_2-COOH \\ \phantom{CH_2=CH-}CH_3 \end{array} \qquad I$$

durch Umsetzung von

a) Verbindungen der Formel V

$$\begin{array}{lllllll} & & & CH_3 & & & \\ CH_2 & - & CH & - \overset{|}{\underset{|}{C}} - & CH=CCl_2 & & V \\ R^3 & & R^4 & CH_3 & & & \end{array}$$

in welcher

$R^3$ für Chlor und

$R^4$ für Wasserstoff oder $R^3$ und $R^4$ gemeinsam eine Doppelbindung bilden,

mit Alkoholaten der Formel IV

$$R^1-(O-\overset{\displaystyle R^2}{\overset{|}{C}H}-CH_2-)_n-OM \qquad IV,$$

in welcher

$R^1$ für Wasserstoff, Alkyl oder M,

$R^2$ für Wasserstoff und Alkyl,



BAD ORIGINAL

M für Alkalimetall und

n für eine ganze Zahl von 1 bis 6 steht,

bei Temperaturen von 100 bis 250°C und nachfolgende Hydrolyse.

Zwar ist die Umsetzung von Vinylidenchlorid mit Alkoholaten der Formel IV zu Ketenacetalen und Orthoessigsäureestern in guten Ausbeuten beschrieben (J. Org. Chem. 29, 2773 (1964) u. J. Org. Chem. 30, 3926 (1965)), doch bei substituierten Vinylidenchloriden wie dem 1,1-Dichlor-1-propen wird nur eine Ausbeute von 8,3 % erreicht (J. Org. Chem. 29, 2773 (1964)).

Es ist deshalb als ausgesprochen überraschend zu bezeichnen, daß nach dem erfindungsgemäßen Verfahren aus den substituierten Vinylidenchloriden der Formel V 3,3-Dimethyl-4-pentensäure in guten Ausbeuten erhalten wird.

Das erfindungsgemäße Verfahren weist neben der allgemeinen Verfügbarkeit der Ausgangsstoffe eine Reihe weiterer Vorteile auf. Die Vorprodukte der 3,3-Dimethyl-4-pentensäure sind aus Isopren einfach und gut zugänglich und deshalb preiswert. Ihre Umsetzung wird mit einfachen Reagenzien wie Alkoholaten durchgeführt und liefert in einem Eintopfverfahren direkt 3,3-Dimethyl-4-pentensäure in guten Ausbeuten.



BAD ORIGINAL

Die Verbindungen der Formel V sind neu. Es handelt sich um 1,1-Dichlor-3,3-dimethyl-1,4-pentadien der Formel II

$$CH_2{=}CH{-}C(CH_3)_2{-}CH{=}CCl_2 \qquad II$$

und um 3,3-Dimethyl-1,1,5-trichlor-1-penten der Formel III

$$Cl{-}CH_2{-}CH_2{-}C(CH_3)_2{-}CH{=}CCl_2 \qquad III.$$

Sie werden aus dem bekannten 1,3-Dichlor-3-methylbutan, dem HCl-Addukt an Isopren, durch Umsetzung mit Vinylidenchlorid unter Aluminiumchlorid-Katalyse zu III und Chlorwasserstoffeliminierung mittels Kaliumfluorid oder Chinolin zu II gewonnen.

$$Cl{-}CH_2{-}CH_2{-}\underset{CH_3}{\overset{CH_3}{C}}{-}Cl + CH_2{=}CCl_2 \xrightarrow[-HCl]{AlCl_3} Cl{-}CH_2{-}CH_2{-}\underset{CH_3}{\overset{CH_3}{C}}{-}CH{=}CCl_2$$

III

$$III \xrightarrow[Chinolin]{KF\ oder} CH_2{=}CH{-}\underset{CH_3}{\overset{CH_3}{C}}{-}CH{=}CCl_2$$

II

Es ist überraschend, daß die Addition von 1,3-Dichlor-3-methylbutan an Vinylidenchlorid unter $AlCl_3$-Katalyse direkt in guter Ausbeute zum 3,3-Dimethyl-1,1,5-trichlor-1-penten III führt, während die gleiche Addition in Gegenwart von Eisen(III)-chlorid in schlechter Ausbeute (18 %) nur 3,3-Dimethyl-1,1,1,5-tetrachlorpentan liefert (J. Amer. Chem. Soc. 74, 2885 (1952)).

Der letzte Syntheseschritt des erfindungsgemäßen Verfahrens kann durch folgendes Formelschema wiedergegeben werden:

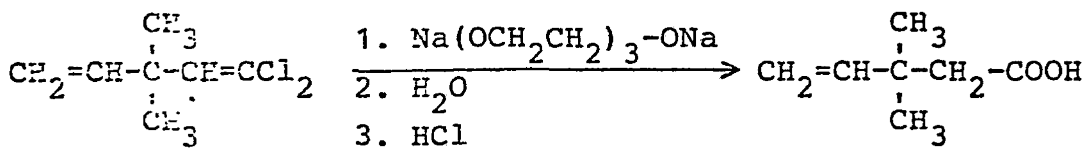


II I

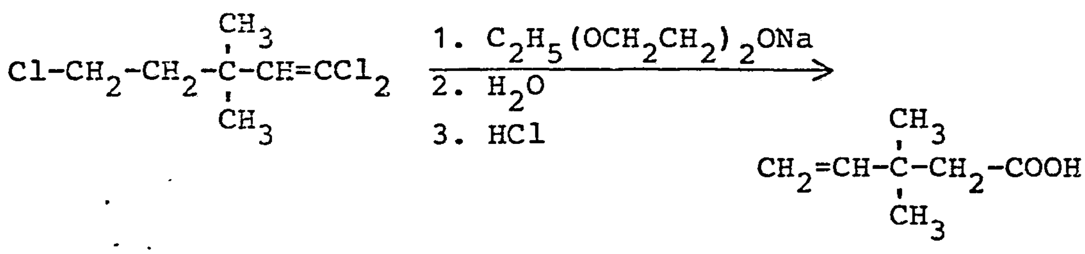


III I

Die Alkoholate sind durch die Formel IV allgemein definiert. $R^1$ steht vorzugsweise für Wasserstoff, niedere Alkylreste mit 1 - 4 C-Atomen oder Na und Kalium, $R^2$ vorzugsweise für Wasserstoff und Methyl. M steht vorzugsweise für Natrium und Kalium. n steht vorzugsweise für 1, 2 und 3. Als Beispiele für die verwendbaren Alkoholate seien im einzelnen die Natrium und Kaliumalkoholate der folgenden Alkohole genannt:

Ethylenglykol, Ethylenglykolmonomethylether, Ethylenglykolmonoethylether, Ethylenglykolmonobutylether, Diethylenglykol, Diethylenglykolmonomethylether, Diethy-



BAD ORIGINAL

lenglykolmonoethylether, Diethylenglykolmonoisopropylether, Diethylenglykolmonobutylether. Diethylenglykolmono-t-butylether, Triethylenglykol, Triethylenglykolmonomethylether, Triethylenglykolmonoethylether, Triethylenglykolmonoisobutylether, Tetraethylenglykol, Tetraethylenglykolmonomethylether, 1,2-Propandiol.

Die Herstellung der Alkoholate erfolgt in bekannter Weise durch Auflösen der Alkalimetalle in den Alkoholen, durch Umsetzung von Alkalimetallhydrid mit den Alkoholen, durch Entwässerung der Alkalihydroxidlösungen in Alkoholen oder durch Auflösen von niederen Alkalialkoholaten in den beschriebenen Alkoholen und Abdestillieren des niederen Alkohols.

Als Verdünnungsmittel kommen die Alkohole der verwendeten Alkoholate, Ether wie Dioxan, Diethylenglykoldimethylether, Triethylenglykoldimethylether, polare aprotische Solventien wie Dimethylformamid, Acetamid, Formamid, N-Methylpyrrolidon, Tetramethylharnstoff, Sulfolan, Dimethylsulfoxid, Hexamethylphosphorsäuretriamid, sowie inerte organische Lösungsmittel wie Xylol, Toluol, Tetralin in Frage. Die Reaktionstemperaturen können in einem größeren Bereich variiert werden. Im allgemeinen arbeitet man zwischen 100°C und 250°C, vorzugsweise zwischen 130°C und 200°C.

Die Umsetzung kann sowohl bei Normaldruck im offenen System als auch unter Druck im Autoklaven ausgeführt werden.



BAD ORIGINAL

Bei der Durchführung des erfindungsgemäßen Verfahrens setzt man 2 bis 6 Äquivalente Alkoholat, vorzugsweise 3 bis 5 Äquivalente, mit einem Äquivalent der Verbindungen II oder III um.

Die nachfolgende Hydrolyse wird nach Zugabe von Wasser oder wäßriger Alkalihydroxidlösung bei Temperaturen von 100 bis 200°C, vorzugsweise bei Rückflußtemperatur, durchgeführt.

Beide Reaktionsschritte können getrennt ausgeführt werden. Vorteilhaft ist die Kombination beider Schritte in einem Einopfverfahren, in dem die Hydrolyse nach Umsetzung mit dem Alkoholat direkt angeschlossen wird. Die folgenden Beispiele illustrieren das erfindungsgemäße Verfahren.



BAD ORIGINAL

Beispiel 1

3,3-Dimethyl-1,1,5-trichlor-1-penten (Verbindung III)

Unter Rühren und Kühlen auf -10°C werden in 2300 g 1,1-Dichlor-ethen 20 g Aluminiumchlorid gelöst. Darauf werden innerhalb von 3 Stunden 1286 g 1,3-Dichlor-3-methylbutan zugetropft und gleichzeitig in Abständen von 15 Minuten jeweils weitere 3 g Aluminiumchlorid zudosiert, wobei durch Kühlung eine Reaktionstemperatur zwischen 0° und +5°C eingehalten wird. Nach Beendigung der Reaktion werden 60 ml Essigsäure zum Reaktionsgemisch zugetropft. Danach wird das Produktgemisch über $Na_2SO_4$ filtriert und anschließend in eine Destillationsapparatur eindosiert, wobei die Sumpftemperatur auf 120°C gehalten und ein Druck von 1 mbar eingehalten wird; das Destillat wird durch eine Trockeneis/Aceton-Mischung gekühlt und kondensiert. Anschließend wird das Rohdestillat an einer Vigreux-Kolonne im Vakuum fraktioniert.

Man erhält 1650 g (= 90 % d. Th.) 3,3-Dimethyl-1,1,5-trichlor-1-penten.
$Kp_{0,1}$ 59 - 63°C.

Beispiel 2

3,3-Dimethyl-1,1-dichlor-1,4-pentadien (Verbindung II)

2200 ml Sulfolan (wasserfrei), 450 g Kaliumfluorid (ge-



BAD ORIGINAL

trocknet) und 760 g 3,3-Dimethyl-1,1,5-trichlor-1-penten werden 2 Stunden auf 200°C unter Rühren erhitzt. Dann wird an einer Kolonne im Wasserstrahlpumpenvakuum das Reaktionsgemisch in ein Produktgemisch vom Siedebereich 20°C bis 130°C/15 mbar und eine höhersiedende Fraktion (hauptsächlich Sulfolan) getrennt. Das Destillat vom Siedebereich 20°C bis 130°C/15 mbar wird anschließend an einer 1,50 m langen Vigreux-Kolonne fraktionierend destilliert. Man erhält neben größeren Anteilen von unverändertem Ausgangsmaterial und von 1,1-Dichlor-5-fluor-3,3-dimethyl-1-penten ($Kp_{15}$ 61 - 64°C) 122 g 3,3-Dimethyl-1,1-dichlor-pentadien-1,4, $Kp_{15}$ 44 - 48°C.

Beispiel 3

1,1-Dichlor-3,3-dimethyl-1,4-pentadien (Verbindung II)

Zu 1 l Chinolin wird bei 225 - 230°C langsam 201,5 g (1 Mol) 3,3-Dimethyl-1,1,5-trichlor-1-penten eingetropft und über Kopf einer Kolonne gleichzeitig Destillat abgenommen. Man steigert die Temperatur in Kolben bis zum Sieden des Chinolins und isoliert insgesamt 126 g Destillat, das nochmals fraktioniert wird. Bei $Kp_{20}$ 49 - 53°C erhält man 121 g (0,73 Mol, 73 %) 1,1-Dichlor-3,3-dimethyl-1,4-pentadien.



BAD ORIGINAL

Beispiel 4

3,3-Dimethyl-4-pentensäure

9,2 g (0,4 g Atom) Natrium werden im Gemisch aus 30 ml Triethylenglykol und 70 ml Triethylenglykoldimethylether gelöst. Bei 200°C tropft man 16,5 g (0,1 Mol) 1,1-Dichlor-3,3-dimethyl-1,4-pentadien zu und erhitzt 8 Stunden auf 200°C. Nach Zugabe von 3,6 g (0,2 Mol) Wasser werden weitere 8 Stunden unter Rückfluß erhitzt. Dann wird mit Wasser verdünnt und mit Methylenchlorid extrahiert. Die alkalische, wäßrige Phase wird anschließend mit Salzsäure sauer gestellt und die Dimethylpentensäure der Formel I mit Methylenchlorid ausgeschüttelt. Aus dieser Lösung erhält man durch Destillation ($Kp_{16}$ 100 - 106°C) 10,4 g (81 %) 3,3-Dimethyl-4-pentensäure.

NMR ($CDCl_3$): $\delta$ 1,15 (s, 6 H); 2,3 (s, 2 H); 4,8 - 6,2 ($-CH=CH_2$) 9,8 (s, 1 H).

Beispiel 5

In die Lösung von 6,9 g (0,3 g Atom) Natrium in 100 ml abs. 2-Ethoxyethanol tropft man unter Rückfluß 16,5 g (0,2 Mol) Dichlordimethylpentadien II und erhitzt 10 Stunden unter Rückfluß. Nachdem 20 ml 10 % Natronlauge zugegeben sind, wird noch 6 Stunden unter Rückfluß erhitzt. Die entsprechende Aufarbeitung wie in Beispiel 4 beschrieben, liefert 9,5 g (74 %) 3,3-Dimethyl-4-pentensäure.



BAD ORIGINAL

Beispiel 6

Aus 12 g (0,3 g Atom) und 35,4 g (0,3 Mol) 2-Butoxyethanol wird in 100 ml Xylol das Kaliumalkoholat bereitet und dann mit 16,5 g (0,1 Mol) Dichlordimethylpentadien II 12 Stunden unter Rückfluß erhitzt. Nach Zugabe von 9 ml $H_2O$ werden weitere 12 Stunden unter Rückfluß erhitzt. Die gleiche Aufarbeitung wie im Beispiel 4 führt zu 9,0 g (70 %) Dimethyl-4-pentensäure I.

Beispiel 7

600 ml 30 % Natriummethylatlösung werden zu 1 l abs. Diethylglykolmonoethylether gegeben und das Methanol anschließend im Vakuum abgezogen. Bei 200°C tropft man 165 g (1 Mol) Dichlordimethylpentadien II zu, erhitzt 8 Stunden unter Rückfluß, hydrolysiert mit 32 ml Wasser 10 Stunden bei Rückflußtemperatur und isoliert nach üblicher Aufarbeitung 92 g (72 %) Dimethyl-4-pentensäure I.

Beispiel 8

3,3-Dimethyl-4-pentensäure I aus III.

320 g (8 Mol) NaOH werden in 3 l Diethylenglykolmonoethylether gelöst und durch Abdestillieren von 1 l Lösungsmittel bei 20 mbar entwässert. Dann tropft man bei 200°C 403 g (2 Mol) 3,3-Dimethyl-1,1,5-trichlor-1-penten zu, erhitzt 8 Stunden unter Rückfluß und nach



BAD ORIGINAL

Zugabe von 72 ml Wasser weitere 12 Stunden unter Rückfluß. Die entsprechende Aufarbeitung wie im Beispiel 4 beschrieben, ergibt 187 g (73 %) Dimethyl-4-pentensäure I.

Beispiel 9

80 g (2 Mol) KOH werden in 800 ml Diethylenglykolmonobutylether gelöst und bei 20 mbar azeotrop entwässert. Bei Rückflußtemperatur gibt man 101 g (0,5 Mol) Dimethyl-trichlorpenten III zu, erhitzt 10 Stunden auf 200°C und hydrolysiert mit 20 ml Wasser in 12 Stunden unter Rückfluß. Die übliche Aufarbeitung führt zu 43,5 g (68 %) Dimethyl-4-pentensäure I.

Beispiel 10

3,3-Dimethyl-4-pentensäuremethylester

In die Lösung von 256 g (2 Mol) Dimethyl-4-pentensäure in 1 l abs. Methanol wird bei 0°C Chlorwasserstoffgas bis zu Sättigung eingeleitet. Nach 12 Stunden arbeitet man mit Methylenchlorid und Wasser auf. Die Fraktionierung über eine Kolonne liefert 262 g (92 %) 3,3-Dimethyl-4-pentensäuremethylester ($Kp_{18}$ 50°C).



BAD ORIGINAL

Patentansprüche

1. Verfahren zur Herstellung von 3,3-Dimethyl-4-pentensäure der Formel

$$\begin{array}{c} \phantom{CH_2=CH-}CH_3\phantom{-CH_2-COOH} \\ \phantom{CH_2=CH-}|\phantom{-CH_2-COOH} \\ CH_2=CH-C-CH_2-COOH \\ \phantom{CH_2=CH-}|\phantom{-CH_2-COOH} \\ \phantom{CH_2=CH-}CH_3\phantom{-CH_2-COOH} \end{array} \qquad I$$

durch Umsetzung von Verbindungen der Formel V

$$\begin{array}{lllll} & & CH_3 & & \\ & & | & & \\ CH_2- & CH- & C- & CH=CCl_2 & \\ | & | & | & & \\ R^3 & R^4 & CH_3 & & \end{array} \qquad V$$

in welcher
$R^3$ für Chlor und
$R^4$ für Wasserstoff stehen; oder $R^3$ und $R^4$ gemeinsam eine Doppelbindung bestehen,

mit Alkoholaten der Formel IV

$$R^1-(\overset{\displaystyle R^2}{\overset{|}{O CH}}-CH_2-)_n OM \qquad IV$$

in welcher

$R^1$ für Wasserstoff, Alkyl oder M,
$R^2$ für Wasserstoff und Alkyl,

M für Alkalimetall und

n für eine ganze Zahl von 1 bis 6 steht,

bei Temperatur von 100 - 250°C und nachfolgende Hydrolyse.

2. Verfahren zur Herstellung von 3,3-Dimethyl-4-pentensäure der Formel I

$$CH_2=CH-\overset{CH_3}{\underset{CH_3}{C}}-CH_2-COOH \qquad I$$

dadurch gekennzeichnet, daß man 1,3-Dichlor-3-methyl-butan mit 1,1-Dichlorethen unter Verwendung von $AlCl_3$ als Katalysator umsetzt und das erhaltene 3,3-Dimethyl-1,1,5-trichlor-1-penten der Formel III

$$Cl-CH_2-CH_2-\overset{CH_3}{\underset{CH_3}{C}}-CH=CCl_2 \qquad III$$

(a) nachfolgend mit Alkoholaten umsetzt und anschließend hydrolysiert,

(b) nach Eliminierung von HCl zu 1,1-Dichlor-3,3-dimethyl-1,4-pentadien der Formel II

$$CH_2=CH-\overset{CH_3}{\underset{CH_3}{C}}-CH=CCl_2 \qquad II$$

diese Verbindung mit Alkoholaten umsetzt und anschließend hydrolysiert.

3. 1,1-Dichlor-3,3-dimethyl-1,4-pentadien der Formel II.

4. 3,3-Dimethyl-1,1,5-trichlor-1-penten der Formel III.

Europäisches Patentamt

**EUROPÄISCHER RECHERCHENBERICHT**

**0045426**

Nummer der Anmeldung

EP 81 10 5675

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| P | CHEMICAL ABSTRACTS, Band 93, Nr. 9, 1. September 1980, Seiten 602-603, Nr. 94833u<br>Columbus, Ohio, U.S.A.<br>Y. DING et al.: "A modified method for synthesis of ethyl (±)cis, trans-2,2-dimethyl-3-(2',2'dichlorovinyl)cyclopropanecarboxylate"<br>& HUA HSUEH HSUEH PAO 1980, 38(1), 89-94<br>* Zusammenfassung *<br>-- | 1,2,4 | C 07 C 51/00<br>57/03<br>21/19<br>21/04<br>17/26<br>17/34<br>69/533 |
| A | DE - A - 2 728 926 (I.C.I.)<br>* Ansprüche 1,4 *<br>-- | 1 | RECHERCHIERTE SACHGEBIETE (Int Cl.³)<br>C 07 C 51/00<br>51/093<br>57/03<br>43/303<br>43/313<br>41/52<br>21/19<br>21/04 |
| A | CHEMISTRY LETTERS, Nr. 6, Juni 1980, Seiten 651-654<br>The Chemical Society of Japan Tokyo, JP.<br>SEI-ICHI HAYASHI et al.: "Defluorination reactions of gem-difluoro- and monofluoroolefins" Novel methods for one-carbon homologations of carbonyl compounds leading to aldehydes, carboxylic acids, and esters"<br>* Insgesamt *<br>------ | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| ✓ | Der vorliegende Recherchenbericht wurde für alle Patentansprüche erstellt. | | |

| Recherchenort | Abschlußdatum der Recherche | Prüfer |
|---|---|---|
| Den Haag | 28-10-1981 | KLAG |